# NEUE EUROPÄISCHE PATENTSCHRIFT

(11) **EP 2 612 126 B2**
(45) Veröffentlichungstag und Bekanntmachung des Hinweises auf die Entscheidung über den Einspruch: **18.08.2021**
(45) Hinweis auf die Patenterteilung: 30.05.2018
(21) Anmeldenummer: 11757805.4
(22) Anmeldetag: 01.09.2011
(51) Int. Cl.: G01M 3/00, G01M 3/20, G01N 25/20, G01N 33/00

(54) **LECKSUCHGERÄT**
LEAK DETECTOR
APPAREIL DE DÉTECTION DE FUITES

(30) Priorität: 20.10.2010 DE 102010048982; 03.09.2010 DE 102010044222
(43) Veröffentlichungstag der Anmeldung: 10.07.2013
(73) Patentinhaber: Inficon GmbH, 50968 Köln (DE)
(72) Erfinder: WETZIG, Daniel, 50999 Köln (DE); DALTON, Scott, Farmington New York 14425 (US); HOFFMANN, Daniel, Irwin Pennsylvania 15642 (US); JACKSON, Walwyn Jr., Syracuse New York 13207 (US)
(74) Vertreter: dompatent von Kreisler Selting Werner - Partnerschaft von Patent- und Rechtsanwälten mbB
(86) Internationale Anmeldenummer: PCT/EP2011/065110
(87) Internationale Veröffentlichungsnummer: WO 2012/028685

(56) Entgegenhaltungen:
- EP-A1- 2 042 849
- DE-A1- 19 735 250
- DE-A1- 19 813 432
- DE-A1-102005 021 909
- DE-A1-102006 047 856
- DE-A1-102008 013 455
- JP-A- H10 213 516
- US-A- 3 786 675

## Beschreibung

Die Erfindung betrifft ein Lecksuchgerät zum Detektieren mindestens einer Gaskomponente in einem angesaugten Gas.

Zur Dichtheitsprüfung mit dem Prüfgas Helium erfolgt der Heliumnachweis in kommerziellen Geräten in der Regel mit einem Massenspektrometer, für dessen Betrieb die Erzeugung eines Hochvakuums erforderlich ist. Eine andere Nachweismethode benutzt eine selektiv für Helium durchlässige Membran, welche einen Hohlraum abschließt, in welchem sich ein Drucksensor befindet. In dem Hohlraum entsteht ein Druck, der dem Partialdruck von Helium in der umgebenden Atmosphäre entspricht. Die Membran erfordert in der Regel eine Beheizung. Ein derartiger Sensor wird als Partialdrucksensor in Wise-Technology bezeichnet. Er wird von der Firma INFICON GmbH hergestellt. Schnüffellecksucher mit Wise-Technology sind beschrieben in DE 10 2005 021 909 A1 und in DE 10 2006 047 856 A1. Derartige Sensoren in Form von Massenspektrometern oder Partialdrucksensoren haben eine hohe Empfindlichkeit, sind allerdings anfällig gegen zu hohe Konzentrationen des Prüfgases. Der Nachweis von Helium mit Wise-Technology ist auf etwa 0,5 mbar Partialdruck begrenzt. Bei Groblecks muss der Sensor vor hohen Konzentrationen geschützt werden. Nach einer groben Verseuchung ist das Gerät für viele Sekunden blind, so dass der Anwender seine Dichtheitsprüfung nicht fortsetzen kann. Insbesondere ist es nicht möglich, eine Grobleckstelle zu lokalisieren. Wird mit einer Schnüffelsonde die Umgebung des Groblecks erreicht, schaltet das System den Sensor zum Schutz blind. In ähnlicher Weise sind auch andere Sensortypen anfällig gegen Verseuchung oder Sättigung. Auch bei Erreichen einer Sättigungsgrenze sind Konzentrationsmessungen nicht mehr möglich.

Ein anderer Sensor vom Typ eines sättigbaren gasselektiven Sensors ist das Massenspektrometer. Das Massenspektrometer hat eine sehr hohe Empfindlichkeit und Gasselektivität, benötigt allerdings für seine Funktion ein Hochvakuum, also einen sehr niedrigen Wert des Totaldrucks an seinem Messeingang. Steigt der Totaldruck über den zulässigen Grenzwert an, so gerät das Massenspektrometer in den Sättigungsbereich.

Der Erfindung liegt die Aufgabe zugrunde, ein Lecksuchgerät von hoher Empfindlichkeit derart weiterzubilden, dass der Messbereich zu höheren Konzentrationen bzw. zu höheren Totaldrücken hin erweitert wird.

DE 2008 013 455 A1 beschreibt einen Totaldrucktransmitter mit zwei Sensorelementen, die in Abhängigkeit von einem Druckschwellwert aktiviert beziehungsweise deaktiviert werden. Ein sättigbarer gasselektiver Sensor, der in einen Blindzustand gesteuert werden kann, wird nicht beschrieben.

JPH10213516A *beschreibt einen massenspektrometrischen Heliumleckdetektor mit einer einen Prüfling evakuierenden Turbomolekularpumpe und einem zusätzlichen Heliumgassensor.*

Eine erste Variante des Lecksuchgeräts nach der Erfindung ist durch den Patentanspruch 1 definiert.

Erfindungsgemäß ist ein erster Sensor vom Typ eines sättigbaren gasselektiven Sensors vorhanden. Zusätzlich ist ein zweiter Sensor vom Typ eines Wärmeleitfähigkeitssensors vorgesehen. Der erste Sensor dient zur Messung niedriger Konzentrationen und der zweite Sensor übernimmt die Messung bei höheren Konzentrationen, bei denen der erste Sensor nicht mehr funktionsfähig ist.

Leckdetektoren mit Wärmeleitfähigkeitssensor sind bekannt. Beispiele solcher Sensoren sind beschrieben in US 3,020,746, US 3,786,675 und JP 09292302 A. Die Sensoren haben einen temperaturabhängigen Widerstand, der in einer Messbrücke entfalten und in einem Gas oder Gasstrom angeordnet ist. Der Gasstrom oder das Gas führt Wärme von dem Widerstand ab. Je größer die Dichte des Gases ist, umso größer ist die Wärmeabfuhr von dem Widerstand. Im Allgemeinen wird die Heizleistung gemessen, die erforderlich ist, um eine konstante Temperatur des Widerstandes einzuhalten. Hierdurch wird die Größe der Wärmeleitfähigkeit des Gases angegeben. Wenn das strömende Gas Luft ist, welches Helium enthält, verringert sich die Dichte des Gesamtgases mit größer werdendem Heliumanteil. Dadurch verringert sich auch die Wärmeleitfähigkeit. Wärmeleitfähigkeitssensoren haben eine geringe Gasselektivität. Sie sind aber nicht sättigungs- oder verseuchungsabhängig und funktionieren selbst bei hohen Konzentrationen einer Gaskomponente in einem Umgebungsgas. Allerdings ist die Messempfindlichkeit begrenzt.

Die Definition, dass der erste Sensor vom Typ eines sättigbaren gasselektiven Sensors ist, ist dahingehend zu verstehen, dass der Sensor oberhalb einer bestimmten Konzentration oder bestimmten Partialdrucks der Gaskomponente kein brauchbares quantitatives Messergebnis mehr liefert. Dies schließt den Fall der Verseuchung ein. Verseuchung tritt bei hohen Konzentrationswerten auf. Nach einer groben Verseuchung ist das Detektorsystem für viele Sekunden blind, so dass der Anwender die Dichtheitsprüfung nicht fortsetzen kann. Insbesondere ist es nicht möglich, eine Grobleckstelle zu lokalisieren. Wird mit der Schnüffelsonde die Umgebung des Groblecks erreicht, schaltet das System den Sensor zum Schutz blind.

*Der Sensor vom Typ eines sättigbaren gasselektiven Sensors kann ein Wise-Sensor sein.*

Da der zweite Sensor als Wärmeleitfähigkeitssensor nicht anfällig gegen zu hohe Konzentrationswerte ist, kann er permanent eingeschaltet sein. Bei niedrigen Konzentrationen sind beide Sensortypen aktiv und bei höheren Konzentrationen wird der erste Sensor blind geschaltet. Die Steuerung der Blindschaltung kann sowohl in Abhängigkeit von Messwerten des ersten Sensors als auch von Messwerten des zweiten Sensors erfolgen.

Die Realisierung des Blindzustandes des ersten Sensors *erfolgt im Falle eines Wise-Sensors dadurch,* dass der Heizstrom durch die gasselektive Membran hindurch unterbrochen wird, so dass die Membran abkühlt und undurchlässiger wird.

Die Erfindung ist bei Schnüffellecksuchern anwendbar, bei denen Gas in eine handgeführte Schnüffelsonde eingesaugt wird. Der zweite Sensor kann an der Schnüffelsonde oder in einem Hauptgerät angeordnet sein, mit welchem die Schnüffelsonde über eine flexible Leitung verbunden ist.

Als Wärmeleitfähigkeitssensoren eignen sich beispielsweise der Sensor AWM 2300 von Hamamatsu oder der Sensor TCS208F3 der Firma Gerhard Wagner.

Mit dem erfindungsgemäßen Lecksuchgerät können auch Kältemittel nachgewiesen werden, wie sie in Klimaanlagen oder Kühlschränken verwendet werden. Kältemittel weisen eine geringere Leitfähigkeit als Luft auf und sind somit über das gegenüber Luft entgegengesetzte Vorzeichen im Signal eines Wärmeleitfähigkeitssensors von Helium bzw. Wasserstoff unterscheidbar.

*Bei einer Variante des Lecksuchgeräts, die nicht Teil der Erfindung ist,* erfolgt eine Umsteuerung von dem ersten auf den zweiten Sensor in Abhängigkeit von dem Totaldruck. Ein derartiges Lecksuchgerät eignet sich in Verbindung mit einem ersten Sensor, der totaldruckempfindlich ist. Hierbei deckt der zweite Sensor den Bereich oberhalb eines kritischen Totaldruckes ab, während der erste Sensor für die Feinmessung benutzt wird. Der zweite Sensor kann zusätzlich so ausgebildet sein, dass er zugleich den Totaldruck misst. In diesem Fall kann der zweite Sensor die Steuerung des ersten Sensors in den Blindzustand bewirken. Alternativ ist es aber auch möglich, einen Totaldruckmesser vorzusehen, der unabhängig von jedem der beiden Sensoren ist und diese steuert.

Es ist auch möglich, mit beiden Sensoren gleichzeitig zu arbeiten, wobei die Quantitätsmessung durch den Wärmeleitfähigkeitssensor erfolgt und die Qualitätsmessung (Gasart-Bewertung) durch einen Partialdrucksensor.

Im Folgenden werden unter Bezugnahme auf die Zeichnungen Ausführungsbeispiele der Erfindung näher erläutert: Es zeigen:
- Figur 1: eine schematische Darstellung eines Schnüffellecksuchers *nach der Erfindung,*
- Figur 2: eine schematische Darstellung eines Leckprüfgerätes mit Prüfkammer, *das nicht Teil der Erfindung ist,*
- Figur 3: ein Diagramm der Messbereiche der beiden Sensortypen und
- Figur 4: eine schematische Darstellung einer Ausführungsform, *die nicht Teil der Erfindung ist.*

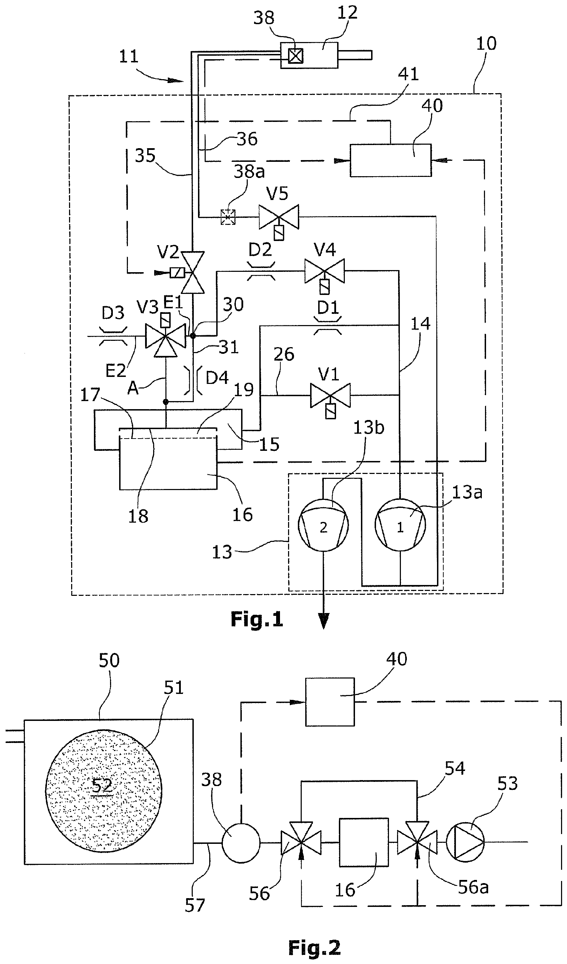

Bei dem Schnüffellecksucher nach Fig. 1 ist ein Grundgerät 10 vorgesehen, das über ein Ventil V2 mit einer Schnüffelsonde 12 verbunden ist. Die Schnüffelsonde 12 kann von Hand geführt werden, um das Testobjekt auf Leckstellen zu untersuchen, aus denen Testgas austritt.

Das Grundgerät 10 enthält eine Vakuumpumpe 13, bei der es sich im vorliegenden Beispiel um eine zweistufige Pumpe mit den Pumpstufen 13a und 13b handelt, die als Membranpumpen ausgeführt sind. Die Vakuumpumpe erzeugt einen Enddruck von etwa 3 mbar.

Von der Vakuumpumpe 13 führt eine Vakuumleitung 14 zu dem Saugraum 15. Der Saugraum 15 ist vor dem Prüfgassensor 16 gebildet. Die Wände des Saugraums 15 schließen an das Gehäuse des Prüfgassensors 16 an. Die Sensorfläche 17 des Prüfgassensors 16 wird von dem Saugraum 15 umschlossen. Innerhalb des Saugraums 15 befindet sich eine Gasführungsplatte 18, die der Sensorfläche 17 mit Abstand gegenüberliegt und parallel zu dieser angeordnet ist. Die Schnüffelleitung 11 mündet in den Gasführungsraum 19. Dieser weist an entgegengesetzten Enden seitliche Öffnungen 20 auf, durch die das Gas in den Saugraum 15 eintreten kann. Der Gasführungsraum 19 bewirkt eine Verteilung des Gases vor der Sensorfläche 17.

Der Prüfgassensor 16 ist in der Weise ausgebildet wie der Sensor, der in DE 100 31 882 A1 beschrieben ist. Die Sensorfläche 17 besteht aus einer selektiv für Helium durchlässigen, elektrisch oder durch Wärmestrahlung beheizten Membran. Im Übrigen enthält der Prüfgassensor 16 einen Penning-Drucksensor oder einen anderen Drucksensor, der ein elektrisches Signal erzeugt, welches den Druck in dem durch eine Quarzmembran abgeschlossenen Gehäuse angibt. Aus diesem Druck wird das Signal für die detektierte Menge an Testgas abgeleitet.

Die Vakuumleitung 14 enthält zwischen der Vakuumpumpe 13 und dem Saugraum 15 eine erste Drossel D1, die die Saugleistung für den NormalBetriebsmodus bestimmt. Die erste Drossel D1 ist durch eine Bypassleitung 26 überbrückt, die ein Ventil V1 enthält.

In einer Lufteinlassleitung befindet sich eine Drossel D3. Das Ventil V3 verbindet entweder den Einlass E1 oder den Einlass E2 mit dem Auslass A. Der Einlass E1 ist mit einem Flussteiler 30 verbunden, welcher durch eine Leitung 31 mit dem Einlass des Prüfgassensors 16 verbunden ist. Die Leitung 31 enthält eine Drossel D4.

Von dem Flussteiler 30 führt ein anderer Weg über eine Drossel D2 und ein Ventil V4 zu der Vakuumleitung 14. Die Drosseln D2 und D4 sind so aufeinander abgestimmt, dass der Fluss durch D2 wesentlich größer ist als derjenige durch D4. Der Fluss durch D2 ist um mindestens 10-mal größer als derjenige durch D4 und insbesondere um mindestens 50-mal. Vorzugsweise beträgt der Fluss durch D2 etwa das Hundertfache des Flusses durch D4.

Die Schnüffelleitung 11, die von der Schnüffelsonde 12 zum Grundgerät 10 führt, enthält eine Messleitung 35, die die Schnüffelsonde mit dem Ventil V2 verbindet und eine Ansaugleitung 36, die über ein Ventil V5 mit dem Einlass der Vakuumpumpe 13 verbunden ist. Die Ansaugleitung 36 verfügt über eine wesentlich höhere Saugleistung als die Messleitung 35. Beispielsweise beträgt die Strömungsrate des angesaugten Gases durch die Messleitung 300sccm und die Strömungsrate durch die Ansaugleitung 36 2700sccm. Die Ansaugleitung 36 dient der Erhöhung der Abstandsempfindlichkeit des Schnüffellecksuchers, indem wesentlich mehr Gas angesaugt wird als im Falle der Messleitung. Durch Abschalten der Ansaugleitung wird die Messempfindlichkeit erhöht. Erfindungsgemäß ist zusätzlich zu dem ersten Sensor 16, der hier als Wise-Technology-Sensor ausgebildet ist, ein zweiter Sensor 38 vorgesehen, bei dem es sich um einen Wärmeleitfähigkeitssensor handelt. Der zweite Sensor 38 ist in der Schnüffelsonde 12 angeordnet und dort insbesondere in der Ansaugleitung 36. Er kann auch im Grundgerät in einer Position 38a angeordnet sein. Dann ist der zweite Sensor an einer Stelle großen Totaldrucks angeordnet, weil dort der Partialdruck der interessierenden Gaskomponente am größten und damit die Nachweisgrenze am günstigsten ist. Eine andere Möglichkeit der Positionierung des zweiten Sensors ist am Auslass der Vakuumpumpe 13. Hierbei wäre jedoch ungünstig, dass das Signal des zweiten Sensors erst zeitlich nach dem Signal des ersten Sensors auftritt. Vorzugsweise sollte das Signal des Wärmeableitungssensors zeitlich vor dem Signal des ersten Sensors vorliegen.

Die Signale des ersten Sensors 16 und des zweiten Sensors 38 werden einer Steuereinrichtung 40 zugeführt, die über eine Steuerfeitung 41 den ersten Sensor 16 blind schaltet, wenn der erste Sensor oder der zweite Sensor eine Konzentration misst, die oberhalb eines Grenzwertes liegt. Damit wird verhindert, dass der erste Sensor in die Sättigung gerät bzw. die Verseuchungsgrenze überschreitet.

Bei dem Ausführungsbeispiel von Fig. 2 handelt es sich um ein Leckprüfgerät, bei dem eine vakuumdichte Prüfkammer 50 vorgesehen ist, in die ein Prüfling 51 eingebracht wird. Der Prüfling 51 ist mit einem Prüfgas 52 gefüllt. Die Prüfkammer 50 wird evakuiert, so dass im Falle eines Lecks des Prüfkörpers 51 Prüfgas aus dem Prüfkörper austritt. An die Prüfkammer 50 ist eine Vakuumpumpe 53 über eine Saugleitung 57 angeschlossen. Die Saugleitung 57 enthält einen ersten Sensor 16 und einen zweiten Sensor 38. Der erste Sensor ist beispielsweise ein Wise-Technology-Sensor und der zweite Sensor ist ein Wärmeleitfähigkeitssensor. Im Strömungsweg des angesaugten Gases ist der zweite Sensor 38 vor dem ersten Sensor 16 angeordnet. Der erste Sensor 16 ist durch eine Bypassleitung 54 überbrückt, die von den Ventilen 56, 56a geöffnet und geschlossen werden kann.

Das Ventil 56 wird durch ein Steuergerät gesteuert in Abhängigkeit von dem Signal des zweiten Sensors 38. Wenn die vom zweiten Sensor gemessene Prüfgaskonzentration einen Grenzwert übersteigt, werden die Ventile 56 und 56a so umgeschaltet, dass der erste Sensor 16 mit der Bypassleitung 54 überbrückt wird. Dadurch wird der erste Sensor gegen Verseuchung geschützt.

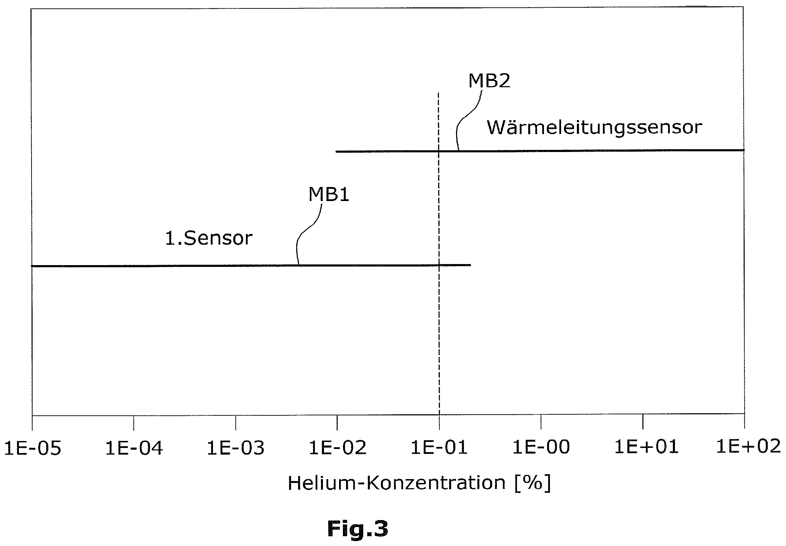

Fig. 3 zeigt ein Beispiel für die Messbereiche des ersten Sensors und des zweiten Sensors am Beispiel der Gaskomponente Helium. Auf der Abszisse ist die Heliumkonzentration aufgetragen. Man erkennt, dass der Messbereich MB1 des ersten Sensors von kleiner 1E-05% (= 10⁻⁴ mbar) bis etwas über 1E-01% (= 1 mbar) reicht, während der Messbereich MB2 des Wärmeleitfähigkeitssensors die ganze Skala oberhalb 1E-02% erfasst. Beide Sensoren ergänzen somit einander.

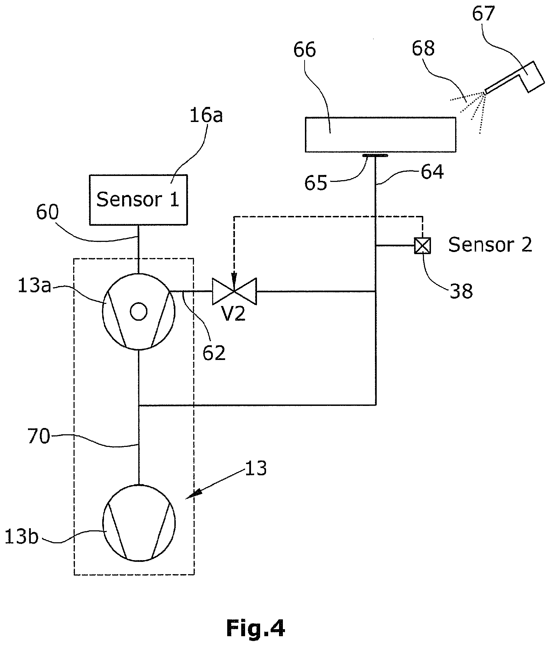

Fig. 4 zeigt ein Ausführungsbeispiel, *das nicht Teil der Erfindung ist,* bei dem ein erster Sensor 16a vorgesehen ist, dessen Funktion von dem Totaldruck an seinem Messeingang 60 abhängt, beispielsweise ein Massenspektrometer. Der Messeingang 60 ist an eine Vakuumpumpe 13 angeschlossen, die eine Hochvakuumpumpe 13a, z.B. eine Turbomolekularpumpe, und eine Vorvakuumpumpe 13b hintereinander enthält. Ein Saugeinlass 62 der Hochvakuumpumpe 13a ist über ein Ventil V2 mit einer Eintrittsleitung 64 verbunden, die einen Anschluss 65 zum Anschließen eines Prüflings 66 aufweist. Der Prüfling 66 ist ein Hohlkörper, der auf Dichtheit zu prüfen ist. Bei dem vorliegenden Ausführungsbeispiel wird mit einem Sprühgerät 67 außerhalb des Prüflings eine Atmosphäre aus einem Prüfgas 68 erzeugt. Das Prüfgas kann von den beiden in dem Leckprüfgerät enthaltenen Sensoren identifiziert werden. Wenn Prüfgas identifiziert wird, ist in dem Prüfling 66 ein Leck vorhanden, durch das Prüfgas 68 eingedrungen ist.

Die Eintrittsleitung 64 ist außerdem an eine Verbindungsleitung 70 angeschlossen, die die beiden Vakuumpumpen 13a und 13b verbindet.

Zwischen den Saugeinlass 62 und die Eintrittsleitung 64 ist ein Ventil V2 geschaltet, das in Abhängigkeit von dem Totaldruck gesteuert ist und in den Sperrzustand gesteuert wird, wenn der Totaldruck über einen Grenzwert ansteigt. Ist das Ventil V2 geschlossen, so ist der erste Sensor 16a von dem Prüfling 66 getrennt, so dass er blindgeschaltet ist.

An die Eintrittsleitung 64 ist der zweite Sensor 38 angeschlossen, bei dem es sich um einen Wärmeleitfähigkeitssensor handelt. Dieser Wärmeleitfähigkeitssensor ist so ausgebildet, dass er bei höheren Drücken vom Totaldruck unabhängig arbeitet. Bei niedrigen Totaldrücken am Messeingang 60 sind beide Sensortypen aktiv und bei höheren Totaldrücken wird der erste Sensor 16a durch Schließen des Ventils V2 blindgeschaltet. Die Messung des Totaldrucks erfolgt bei dem vorliegenden Ausführungsbeispiel an der Eintrittsleitung 64 mit Hilfe des zweiten Sensors 38. Alternativ könnte sie auch am Messeingang 60 des ersten Sensors erfolgen. Es ist auch möglich, für die Totaldruckmessung ein eigenes Messgerät zu verwenden.

## Patentansprüche

1. Lecksuchgerät mit
einem Hauptgerät (10) mit
einer Vakuumpumpvorrichtung (13; 53), durch die Gas angesaugt wird, und
einem ersten Sensor (16) zum Detektieren mindestens einer Gaskomponente in dem angesaugten Gas, wobei der erste Sensor vom Typ eines sättigbaren gasselektiven Sensors ist, und mit
einer mit dem Hauptgerät verbundenen Schnüffelsonde (12),
**dadurch gekennzeichnet,**
**dass** ein zweiter Sensor (38) vom Typ eines Wärmeleitfähigkeitssensors zum Detektieren der Wärmeleitfähigkeit des angesaugten Gases an der Schnüffelsonde oder in dem Hauptgerät, an einer Stelle großen Totaldrucks, vorgesehen ist, und
**dass** Mittel vorgesehen sind, um den ersten Sensor (16) in einen Blindzustand zu steuern, wenn der erste und/oder der zweite Sensor eine Konzentration der Gaskomponente oberhalb eines Grenzwertes feststellt, um dadurch zu verhindern, dass der erste Sensor in Sättigung gerät oder eine Verseuchungsgrenze überschreitet, wobei der erste Sensor (16) den Bereich niedriger Konzentrationen der Gaskomponente und der zweite Sensor (38) den Bereich hoher Konzentrationen abdeckt, bei denen der erste Sensor nicht mehr funktionsfähig ist, und
**dass** der erste Sensor (16) eine beheizbare selektiv gasdurchlässige Membran (17) aufweist, und dass zur Realisierung des Blindzustandes die Membranheizung abgeschaltet wird.

2. Lecksuchgerät nach Anspruch 1, **dadurch gekennzeichnet, dass** eine Schnüffelsonde (12) zum Ansaugen von Gas aus der Atmosphäre vorgesehen ist, und dass der erste Sensor (16) in einer Leitung (31) und der zweite Sensor (38) in einer Ansaugleitung (36) enthalten ist, wobei die Ansaugleitung zur Erhöhung der Abstandsempfindlichkeit direkt von der Schnüffelsonde (12) zu der Vakuumpumpvorrichtung (13) führt und eine höhere Strömungsrate führt als die Messleitung.

3. Lecksuchgerät nach Anspruch 1 oder 2, **dadurch gekennzeichnet, dass** der zweite Sensor (38) an einem Ort mit konstantem Totaldruck von annähernd Atmosphärendruck angeordnet ist, insbesondere in einer Ansaugleitung (36) oder an einem Luftauslass des Lecksuchgerätes.

## Claims

1. A leak detector, comprising
a main device (10) having
a vacuum pump device (13; 53) through which gas is taken in, and
a first sensor (16) for detecting at least one gas component in the gas taken in, the first sensor being a saturable gas-selective sensor type, and comprising
a sniffer probe (12) connected to the main device,
**characterized in that**,
a second sensor (38) of the thermal conductivity sensor type is provided to detect the thermal conductivity of the gas taken in at the sniffer probe or in the main device, at a point of large total pressure, and
means are provided for switching the first sensor (16) to a blind mode when the first and/or the second sensor detects a concentration of the gas component above a limit value in order to prevent that the first sensor is saturated or exceeds a contamination limit, the first sensor (16) covering the range of low concentrations of the gas component and the second sensor (38) covering the range of high concentrations in which the sensor is not functional anymore, and
that the first sensor (16) comprises a heatable, selectively gas-permeable membrane (17), and that the membrane heating is deactivated in order to realize the blind mode.

2. The leak detector according to claim 1, **characterized in that** a sniffer probe (12) is provided for taking in gas from the atmosphere, and that the first sensor (16) is included in a conduit (31) and the second sensor (38) is included in an intake conduit (36), the intake conduit leading directly from the sniffer probe (12) to the vacuum pump device (13) and carrying a higher flow rate than the measuring conduit, so as to increase the distance sensitivity.

3. The leak detector according to claim 1 or 2, **characterized in that** the second sensor (38) is arranged at a position where a constant total pressure prevails that approximates atmospheric pressure, the sensor being arranged in particular in an intake conduit (36) or at an air outlet of the leak detector.

## Revendications

1. Appareil de recherche de fuites, avec
un appareil principal (10), avec
un dispositif de pompe à vide (13 ; 53) à travers lequel du gaz est aspiré, et
un premier capteur (16) destiné à la détection d'au moins une composante gazeuse dans le gaz aspiré, le premier capteur étant du type capteur sélectif de gaz saturable, et avec
une sonde renifleuse (12) raccordée à l'appareil principal,
**caractérisé**
**en ce qu'**il est prévu, dans un emplacement de pression totale élevée, un deuxième capteur (38) du type capteur de conductibilité thermique destiné à la détection de la conductibilité thermique du gaz aspiré sur la sonde renifleuse ou dans l'appareil principal, et
**en ce qu'**il est prévu des moyens pour amener le premier capteur (16) dans un état aveugle quand le premier capteur et/ou le deuxième capteur constate une concentration de la composante gazeuse supérieure à une valeur limite, afin d'empêcher ainsi que le premier capteur se retrouve dans un état de saturation ou dépasse une limite de contamination, le premier capteur (16) couvrant la plage de faibles concentrations de la composante gazeuse, et le deuxième capteur (38) couvrant la plage de concentrations élevées pour lesquelles le premier capteur n'est plus opérationnel, et
**en ce que** le premier capteur (16) comprend une membrane (17) chauffable perméable au gaz de façon sélective, et en ce que le chauffage de membrane est mis hors circuit pour la réalisation de l'état aveugle.

2. Appareil de recherche de fuites selon la revendication 1, **caractérisé en ce qu'**il est prévu une sonde renifleuse (12) destinée à l'aspiration de gaz à partir de l'atmosphère, et **en ce que** le premier capteur (16) est contenue dans une conduite (31) et le deuxième capteur (38) est contenu dans une conduite d'aspiration (36), la conduite d'aspiration conduisant, pour l'augmentation de la sensibilité vis-à-vis de la distance, directement de la sonde renifleuse (12) vers le dispositif de pompe à vide (13), et conduisant un débit d'écoulement plus élevé que la conduite de mesure .

3. Appareil de recherche de fuites selon la revendication 1 ou 2, **caractérisé en ce que** le deuxième capteur (38) est disposé dans un endroit avec une pression totale constante proche de la pression atmosphérique, en particulier dans une conduite d'aspiration (36) ou à la sortie d'air de l'appareil de recherche de fuites.
